# EUROPEAN PATENT APPLICATION

(11) **EP 4 745 221 A1**
(43) Date of publication of application: **20.05.2026**
(21) Application number: 24746771.5
(22) Date of filing: 21.06.2024
(51) Int. Cl.: C12H 1/22, G01N 33/14, C12G 1/00, C12G 3/00

(54) **APPARATUS, SYSTEM AND METHOD FOR MONITORING THE AGING AND/OR MACERATION PROCESSES OF AN ALCOHOLIC BEVERAGE IN A BARREL AND ALCOHOLIC BEVERAGE OBTAINED**

(30) Priority: 13.07.2023 ES 202330588
(71) Applicant: Productos Agrovin, S.A., Alcázar de San Juan 13600 Ciudad Real (ES); Universidad De Valladolid, 47002 Valladolid (ES)
(72) Inventor: NEVARES DOMÍNGUEZ, Ignacio, 47008 Valladolid (ES); DEL ÁLAMO SANZA, María, 47008 Valladolid (ES); JURADO FUENTES, RIcardo, 13600 Ciudad Real (ES)
(74) Representative: Clarke, Modet y Cía., S.L.
(86) International application number: PCT/ES2024/070391
(87) International publication number: WO 2025/012495

(57) **Abstract**

The present invention describes an apparatus, a system, and a method for monitoring the aging and/or maceration processes of an alcoholic beverage in a barrel, and an alcoholic beverage obtained according to said method, comprising an apparatus that hermetically seals the barrel, and a minimum of three sensors, two for environmental conditions and one for distance, that allow monitoring the processes and broadcasting alarms and/or alerts that warn the user whenever an undesired situation occurs.

## Description

The present invention refers to an apparatus, a system, and a method for monitoring the aging and/or maceration processes of an alcoholic beverage in a barrel and to an alcoholic beverage obtained according to said method.

### Prior art

Barrels are constructed of pieces of wood or substantially of wood and/or its derivatives to contain alcoholic beverages. It is a container that is affected by changes in external and internal environmental conditions, since wood and its derivatives are anisotropic and porous materials that change with environmental conditions and when contacted with liquid, which causes changes especially in its dimensions. Wood and its derivatives expand and contract depending on their moisture content and do so differently in the three directions (radial, longitudinal and tangential).

When a barrel is full, the liquid content soaks the inside of the wood, while the outside of the barrel is in contact with the ambient air of its microenvironment. This arrangement means that, depending on the environmental conditions surrounding the barrel, the evaporation of water and ethanol, the main components of alcoholic beverages, can occur to varying degrees. In addition to the fundamental fact that wood is hygroscopic (it gains or loses moisture depending on environmental conditions), there is also another crucial concept to consider in relation to wood and moisture, and that is the link between relative humidity and the wood's equilibrium moisture content or hygroscopic equilibrium moisture, which is called EMC *(Equilibrium Moisture Content).*

The moisture content when the cell walls are completely saturated (all water is bound), and there is no water in the cell lumens is called the fiber saturation point (FSP- *Fiber Saturation Point)* and corresponds to a moisture content between 21 % and 35 % depending on the wood species. The first moisture that soaks into the wood binds to the cells and causes them to change their size. While below the FSP the water is bound, and only when the FSP point is reached and exceeded does the porosity (free space filled with air inside the wood) that the wood has at that moment begin to fill with free water. Thus, for each level of relative air humidity there is a corresponding hygroscopic equilibrium moisture content in the wood and, therefore, whenever the humidity of the wood is below the fiber saturation point, a dimensional variation will occur to adapt to the environmental conditions. Depending on the arrangement of the barrel, the different exposure of the barrel to environmental conditions causes the radial dimensional variation caused by humidity changes to reach up to 4 %, varying the width of the staves and, therefore, the shape of the barrel, as well as the strength of the joint between two staves. These variations influence the actual volume of the barrel, the internal pressure, the rate of evaporation of water and ethanol, as well as the rate of oxygen permeation from the outside of the barrel to the inside where the alcoholic beverage is. Therefore, it is necessary not only to know the conditions of the barrel environment but also the interior conditions, such as relative humidity and pressure and temperature.

With all these factors in play, it is clear that as soon as the barrel is filled, the alcoholic beverage begins to permeate the wood, i.e., it gets into the wood and its porosity, and with it the volume of liquid inside the barrel is reduced, which does not indicate that a loss of alcoholic beverages by evaporation is taking place, this does not indicate a loss of alcoholic beverages through evaporation, since it takes forty days for the beverage that has penetrated the wood to pass through the thickness of the wood staves necessary to initiate the evaporation process. In addition, the impregnation/wetting process of the wood causes part of the volume of alcoholic beverages/beverage to soak into the wood, decreasing its volume and generating a depression, but usually in the first few weeks there is no drop in the level of the liquid inside. Although no drop in liquid level can be observed inside, the container/barrel is changing to adapt to the new volume of liquid/beverage and a depression is being generated. This is because since wood is flexible, as it is impregnated with alcoholic beverages/beverage, each of the parts that make up the barrel can adapt its shape to accommodate this variation in volume, and, therefore, the liquid level can remain the same. Only with the joint knowledge of the interior and exterior conditions of pressure, temperature, and relative humidity is it possible to know the dimensional state of the barrel and to know whether the possible decrease in liquid volume is due to the wetting of the wood or to evaporation caused by the environmental conditions.

This volumetric adaptation occurs up to a certain level at which the barrel can no longer be deformed and a gaseous space begins to appear at the top of the vessel. All this (barrel deformation and formation of the upper gas space) happens when the interior depression is maintained. Only with the joint knowledge of the internal conditions of pressure, temperature, RH, and height of the gas space, and the external conditions of pressure, temperature, and RH, it is possible to know the dimensional state of the barrel and to determine whether the decrease in the volume of liquid/beverage is due to the wetting of the wood and/or evaporation caused by the environmental conditions.

Numerous monitoring systems are currently used to follow the evolution of the parameters of the alcoholic beverage during its time in barrels, these monitoring systems include the use of different sensors.

In the current state of the art, either specific sensors for the parameters of interest or generic sensors specifically calibrated for the different parameters are used. In any case, these sensors are capable of quantifying the corresponding analyte/parameter of the alcoholic beverage contained in the barrel (pH, temperature, alcoholic strength, dissolved gases such as O₂ or CO₂, turbidity, SO₂, acetic acid, tartaric acid, acetaldehyde, total acidity, volatile acidity, color, ...). In addition, other physical data of interest for the aging of the alcoholic beverage in the barrel are also monitored, such as the distance from the bung to the surface of the alcoholic beverage, which is measured with different sensors, both wireless and submersible, which are based on pressure difference.

The filling of alcoholic beverage barrels during aging and/or maceration is a common practice in the alcoholic beverage industry (wine, fortified wine, distilled spirits, beer, etc.). This technique consists of replenishing the volume of alcoholic beverages lost in the barrels due to evaporation and absorption of the alcoholic beverages by the wood. This operation is considered very important in order to maintain the integrity of the aging and/or maceration process and to ensure the quality of the final alcoholic beverage.

Traditionally, it has been considered that the generation of a gaseous space at the top of the barrel, created by the loss of alcoholic beverage caused by the absorption of part of the alcoholic beverages contained in the barrel during its wetting and, in addition, evaporation, has as a consequence the drying of the wood in contact with this gaseous space. In this scenario, where the surface of these wood staves is believed to be dry, it is assumed that there is a high intake of air from the outside to the inside of the barrel to reach the alcoholic beverage. Therefore, the filling of barrels is considered essential to maintain the quality of the alcoholic beverage by preventing excessive oxidation. Thus, by replenishing the volume of alcoholic beverage lost, the exposure of the alcoholic beverage to oxygen is minimized, which helps to preserve the aromatic and taste quality of the alcoholic beverage and prevents the development of possible defects.

Despite the apparent advantages, the filling of barrels also has the disadvantages described below.

The filling of barrels involves the use of an additional quantity of alcoholic beverages to compensate for the loss of volume. In terms of costs, the filling of barrels represents an additional expense for the producer, since it involves the additional consumption of product and associated resources, such as time and personnel dedicated to the task. The frequency of filling the barrels can vary, but on average it is usually done every 1-2 months during the aging and/or maceration period, although it is more frequent during the first month (weekly). The cost of filling the barrels depends on the size of the winery, the number of barrels to be filled, and the volume of alcoholic beverages required for each filling. As an example, considering a medium-sized wine cellar with 100 oak barrels, the average cost per barrel fill can range from 10 to 50 euros, depending on the prices of the wines used for filling. This includes the cost of the alcoholic beverage used and the associated resources, such as the time and personnel involved in the process.

It is important to note that these values are averaged estimates and may vary considerably depending on the specific circumstances of each producer. In addition, some wineries choose to fill the barrels more frequently and with smaller volumes, while others may opt for less frequent filling, but with larger volumes of alcoholic beverages.

On the other hand, the filling of barrels requires proper time management and the corresponding logistics. It is necessary to schedule the filling at appropriate times to avoid prolonged exposure of the alcoholic beverage to oxygen and to ensure a proper balance between the evolution of the alcoholic beverage and the absorption of the wood. The time required to fill the barrels during the aging and/or maceration of the alcoholic beverage can vary according to several factors, such as the size of the winery, the number of barrels to be filled, the availability of personnel, and the winery's internal logistics. An average estimate of the time required to perform each of the stages into which this operation can be divided is given below:
Preparation: Before starting the filling process, it is necessary to prepare the alcoholic beverage that will be used to fill the barrels. This involves the selection of the appropriate alcoholic beverage, its transfer and the preparation of the necessary tools and equipment. This stage can take between 1 and 2 hours, depending on the organization and internal procedures of the winery.
Filling of barrels: The time required to fill each barrel may vary depending on the size of the barrel and the technique used. On average, the estimated time to fill a barrel ranges from 10 to 30 minutes. This includes transferring the alcoholic beverage, checking the filling level, and handling the necessary tools.
Registration and documentation: After filling the barrels, it is important to keep a proper record of each operation. This involves documenting the date, type of alcoholic beverages used, barrel number, and any relevant observations. The time required for this process can vary, but it is estimated to take between 5 and 15 minutes per barrel, depending on the level of detail required and the efficiency of documentation.
Cleaning and organization: Once the filling process is completed, it is necessary to clean and organize the equipment used, as well as the work area. This stage can take between 30 minutes and 1 hour, depending on the number of barrels filled and the complexity of the equipment used.

In summary, considering all the stages involved, it is estimated that the average time required to carry out the process of filling the barrels during the aging and/or maceration of the alcoholic beverage is between 2 and 4 hours for every 10 barrels. This estimate may vary according to the specific circumstances of each winery, such as the size of the winery, the number of barrels to be filled, and the internal organization.

Finally, it should be noted that sometimes the filling of barrels affects the uniformity of the alcoholic beverage, as each barrel may experience a different degree of evaporation and absorption. This results in more or less subtle variations between barrels, which requires careful monitoring and subsequent mixing in order to ensure the consistency and quality of the final alcoholic beverage.

There is, therefore, a need for an apparatus, a system and a method for monitoring the aging and/or maceration processes of an alcoholic beverage in a barrel in which the filling of barrels is eliminated or minimized, reducing costs, time and logistics and, to a greater extent, maintaining the uniformity of the aging and/or macerating alcoholic beverage.

The object of the present invention is an improved apparatus, system, and method for obtaining a solution for at least one of the above-mentioned drawbacks related to the aging and/or maceration of alcoholic beverages, as defined in the claims. It is another object of the invention, an alcoholic beverage obtained by means of the method according to the invention.

### Description of the invention

Throughout the invention and the claims, the word "comprises" and variants thereof are not intended to exclude other technical features, components or steps. In addition, the word "comprising" includes the term "consisting of". To those skilled in the art, other objects, advantages, and features of the invention will be apparent in part from the description and in part from the practice of the invention.

In this document, "a/an" and "the" refer to singular and plural, unless the context clearly implies otherwise. For example, a "barrel" means a barrel or more than one barrel.

The term "alcoholic beverage" refers to, but is not limited to, wine, fortified wine, distilled spirit, beer, and cider, among others, alone and/or in combination with each other and with other products.

In the present invention, "barrel" is understood as any container for containing alcoholic beverages constructed of pieces of wood or substantially of wood and/or its derivatives, whatever their shape, volume, and size.

"Processes of aging and/or maceration of an alcoholic beverage in a barrel" means the extraction, transfer, and integration into said alcoholic beverage of the chemical compounds contained both in the parts of the barrel and in added products, such as, but not limited to, wood alternatives, aromatic herbs, treacle, syrups, and other additives. The added products can be used alone or in combination with other added products.

The liquid added products are mixed and the solids are introduced into the alcoholic beverage in bulk or in textile bags, food plastic bags, or the like, leaving them to act for the necessary time until the chemical compounds are transferred to the alcoholic beverage by means of a series of chemical and/or biological phenomena or transformations, such as, but not limited to, malolactic fermentation.

Unless otherwise defined, all terms used in the description of the invention, including technical and scientific terms, have as their meaning the meaning generally understood by the person skilled in the art in the technical field of the invention.

When a barrel is filled with an alcoholic beverage, the volume of the beverage decreases because some of it is absorbed by the wood, and some evaporates. If the barrel is hermetically sealed, a depression is generated inside the barrel, causing it to deform in order to adapt to the new volume of beverage. Under these conditions, when the limit of deformation of the wood to adapt to the new volume has already been reached, a headspace is generated; the headspace comprising a gaseous volume located at the top of the barrel that is in direct contact with the bung and with the alcoholic beverage. Volume of gas that is in equilibrium with the alcoholic beverage contained in the barrel, this being a representative measurement point of occurs in the alcoholic beverage.

The monitoring of the aging of an alcoholic beverage in a barrel by using an apparatus that produces a hermetic seal on the barrel makes it possible to eliminate or significantly reduce the intervention during the entire aging and/or maceration period.

"Intervention during the aging and/or maceration time" means, in the present invention, the removal of the sensorized apparatus that closes the barrel and the addition of the amount of product and/or additive necessary for the correction of the undesired situation detected.

As a consequence of the elimination and/or reduction of the intervention, there is less evaporation-loss of alcoholic beverages than in the usual processes where the barrels are periodically filled, also influencing the reduction of labor costs.

With the invention, the costs necessary and arising from the filling operation are eliminated for the most part. After careful study of the operation of a barrel during an aging process of an alcoholic beverage it has been found that, surprisingly, the formation of the headspace does not cause the drying of the wood at the top, as is commonly accepted, but rather the saturation of water vapor, and ethanol primarily from that gaseous space prevents the wood, wet when the barrel was filled, from drying out. This fact makes it possible to consider monitoring the aging of the alcoholic beverage with the use of an apparatus that allows the least intervention, or even no intervention at all.

A sensorized apparatus has been developed to monitor the tightness of the barrel seal by controlling the generation of an internal depression, which, when maintained over time, ensures that no air enters from the outside and, therefore, no atmospheric oxygen enters the barrel. The monitoring of the environmental conditions inside the barrel (pressure, temperature, and relative humidity) ensures that the wood of the upper staves maintains its initial state of humidity, thanks to the saturation of the gaseous space with water vapor/ethanol, ensuring not only its physical integrity (no cracks appearing) but also the correct fit between the different parts as the staves maintain their swelling and the consequent tightness of the joints.

Monitoring the changes in headspace volume together with the interior depression allows us to know if there is any modification of the original shape of the barrel, i.e., deformation to adapt to the lower volume of alcoholic beverages due to loss by absorption of alcoholic beverages by the wood and evaporation to the environment.

Unlike the prior art, the present invention focuses on considering the barrel as an active (because it changes intrinsically) and dynamic (because it changes as the environment changes) container, and, therefore, the internal state and the external state of the barrel are monitored in order to define the situation in which it finds itself. The fundamental parameters measured are the environmental conditions inside and outside the barrel, i.e., barometric (absolute) pressure, temperature, and relative humidity. The analysis of these parameters allows us to know the state and also the evolution of the behavior of the barrel as an active container which contains an alcoholic beverage subjected to an aging and/or maceration process.

The (internal-external) pressure difference allows us to know if there is an internal depression caused by the absorption of beverage in the wood and by the evaporation defined by the environmental conditions. As long as the internal pressure remains below the external pressure, no intervention is necessary during the entire aging and/or maceration period.

If, conversely, an overpressure (Pₑₓₜ-Pᵢₙₜ < 0) is detected, it would allow us to know whether it is due to the release of CO₂ by fermentation or to the increase in the volume of the beverage, a phenomenon observed when, after filling the barrel with a liquid at a lower temperature than that of the barrel room, the beverage undergoes an increase in temperature and the corresponding dilatation, as it is tempered to the new conditions.

If an internal temperature (inside the barrel higher than the external temperature (corresponding to the barrel environment) is detected, it may indicate that exothermic reactions are occurring in the beverage that may be indicative of undesirable reactions, such as microbiological contamination. As long as the internal temperature remains below the external temperature, no intervention is necessary during the entire aging and/or maceration period.

On the other hand, variations in the humidity conditions of the barrel's environment cause changes in the EMC, which in turn produce dimensional variations that affect the geometric shape of the barrel and, therefore, the pressure difference between the atmosphere and the internal pressure of the barrel. Since the liquid is practically incompressible, these pressure differences directly affect the headspace pressure. As long as the internal relative humidity exceeds the external relative humidity, an intervention will not be necessary during the entire aging and/or maceration period

The determination of the fundamental parameters of the barrel's internal and external environmental conditions is carried out with an apparatus for monitoring the aging and/or maceration processes of an alcoholic beverage in a barrel that is placed in the mouth or bunghole of the barrels, comprising a bung with an interior space as a headspace, which can be made of rubber or polymeric, or of any other material that achieves a good fit with the bunghole of the barrel, preferably silicone, and a tightening washer that hermetically closes the barrel in order to generate and maintain a headspace in equilibrium with the alcoholic beverage, even when the barrel is completely full. This apparatus incorporates at least three sensors in its own internal space: a sensor of internal environmental conditions and measurement of the partial pressure of dissolved and volatile gases and a distance sensor, both located in the head space of the apparatus in contact with the headspace of the barrel and a sensor of environmental conditions of the microenvironment of the barrel located in the part of the apparatus in contact with the barrel room.

In this situation, knowing the distance from the apparatus to the surface of the beverage, through the distance sensor, the headspace volume can be determined, and the set and the beverage volume-gas volume ratio in the barrel can be established. This set/system is balanced and evolves over time, as the aging and/or maceration of the alcoholic beverage in the barrel develops.

Distance sensors for liquid level measurement can be those of time-of-flight *(ToF - Time-of-Flight)* or ultrasonic, among others. In a preferred embodiment of the invention, the at least one distance sensor is of the time-of-flight based type, which measures the time elapsed from the emission of a wave pulse from the sensor to the time at which the wave pulse returns to the sensor after reflecting off an object or surface. The transmitter of the time-of-flight device emits IR (infrared) waves towards the target object, the wave is reflected upon reaching the object. The distance is calculated using the speed of light in air and the time elapsed between sending and receiving the signal. Advantageously, time-of-flight sensors offer faster readings, with greater accuracy and longer range than ultrasonic distance sensors.

For the different beverage compounds that are volatile, the vapor pressure arises due to the escape of liquid molecules into the gaseous mixture from the headspace of the closed system and varies depending on the temperature variations of a system. The vapor pressure does not depend on the volume of the system or the surface area of the liquid.

The apparatus according to the invention makes it possible to know the partial pressures of the different gases dissolved in the alcoholic beverage by measuring the partial pressure of that gas in the headspace which is in equilibrium with a known volume of liquid/alcoholic beverage. In this way, the dissolved gas content can be determined without touching the alcoholic beverage.

The volatile components of the alcoholic beverage can also be determined by measuring the vapor pressure of a compound in the headspace with which it is equilibrated.

Preferably, the apparatus according to the invention uses the measurement of:
a) the partial pressure in the headspace of the barrel of a gas dissolved in the beverage (CO₂, O₂ and others) to determine its partial pressure as a dissolved gas.
b) the vapor pressure in the headspace of the barrel of a volatile compound of the alcoholic beverage (such as SO₂, ethanol, 4-ethylphenol, acetic acid, ethyl acetate, acetaldehyde, and any other volatile compound of the alcoholic beverage) to determine its concentration in the alcoholic beverage.

For the measurement of the partial pressure of dissolved and volatile gases it is proposed to use at least one sensor that in addition to measuring the environmental conditions of the internal space of the barrel (barometric (absolute) pressure, temperature, and relative humidity of the headspace) has a gas scanner function that can detect volatile organic compounds (VOCs), volatile sulfur compounds (VSCs), and other gases such as carbon monoxide and hydrogen. The sensor preferably integrates artificial intelligence (AI) and can be trained to detect different substances of interest in the aging and/or maceration of an alcoholic beverage, such as, but not limited to, SO₂, ethanol, 4-ethylphenol, acetic acid, ethyl acetate or acetaldehyde, among others.

The arrangement of a gas scanner sensor in the head space of the bung itself, which works as an electronic nose, makes it possible to know the content of different analytes present in the beverage contained in the barrel. The headspace is formed by gases that are in equilibrium with the liquid contained in the barrel, which allows the evaluation of its contents during aging.

Once the gas scanner sensor has been trained for the different alarm thresholds of the different analytes, the system will trigger a warning when it detects that the programmed thresholds have been reached. With this information, the oenologist will make the decision to intervene in the barrel to check, analyze, confirm, correct and/or condition in order to return to the desired situation in the beverage.

Thus, the gas scanner sensor can be trained to know if the wine is being enriched in compounds such as 4-ethyl phenol (due to microbiological drift) which has a great aromatic impact as it is responsible for horse sweat notes, detecting levels above 50 parts per billion of this compound in the wine. This would allow the oenologist to intervene in the early stages of possible microbiological drift, well before reaching amounts that cause unpleasant notes in the beverage (sensory detection limit). In the same way, the headspace gas scanner sensor can detect the increase of another compound such as acetic acid, related to another chemical or microbiological deviation, showing when the wine presents levels above 0.6 grams per liter of acetic acid, as well as 0.1 grams per liter of ethyl acetate or acetaldehyde.

Another compound that can be detected is sulfur dioxide, currently the most important preservative agent used in oenology to maintain and protect wine. Thus, the gas scanner sensor, after specific training, is able to detect when the wine has less than 20 mg/L and therefore, the oenologist must intervene to increase this content and ensure that the beverage is protected in the barrel.

The sensor consists of a system for data acquisition of the conditions inside the barrel, wherein it will be possible to analyze parameters of pressure, level, temperature, gas concentrations (by direct measurement or associated measurement by programming the microchip determined for this purpose), and even pH or redox potentials. In addition, external measurements of the conditions outside the barrel (temperature, pressure, humidity) will be taken.

Each sensor will be programmed to generate specific data packets to be sent to a base station via radio signals on a periodic basis. To avoid excessive battery consumption, a hibernation time will be set for each device, depending on the number of measurements per day.

As a result of the calibration process to which they are subjected, the sensor of internal environmental conditions and measurement of the partial pressure of dissolved and volatile gases allow obtaining qualitative values to control whether the contents of gases and/or volatile compounds are within predefined warning ranges or thresholds.

For external environmental conditions, it is proposed to use at least one sensor that measures barometric pressure, temperature, and relative humidity for the external environment or environmental conditions of the microenvironment of each barrel. Their knowledge will allow us to know the evaporation rate of water and ethanol, the main components of alcoholic beverages, for example, and of many beverages stored in wooden barrels and barrels of other materials.

Advantageously, the at least one sensor for internal environmental conditions and measurement of the partial pressure of dissolved and volatile gases and the at least one distance sensor are assembled together so as to constitute a package of interchangeable sensors, thus facilitating the maintenance of the apparatus in which, in the event of any failure, it is only necessary to change a quick and simple coupling package in said apparatus.

Optionally and for specific applications, the apparatus for monitoring the aging and/or maceration processes of an alcoholic beverage in a barrel may comprise specific sensors of, for example, CO2, SO2, REDOX potential, and others that allow to obtain the quantitative values of the specific substances/characteristics under control.

In another aspect of the invention, the apparatus incorporates a self-contained power source, such as a battery, alkaline batteries, etc.

Also, in another aspect of the invention, the apparatus can perform data transmission by wire or wirelessly. Preferably, the data transmission from the apparatus is performed wirelessly.

Still, in another aspect of the invention, the apparatus may comprise a device for taking samples of the alcoholic beverage contained in the barrel which allows to extract a sample without removing it from the mouth or bunghole of the barrel, keeping the closure of the barrel airtight. Sample intended to check the evolution of the alcoholic beverage, both through sensory analysis or tasting and through a chemical analysis of volatile acidity, sulfur content, pH, etc.

In one embodiment, said device consists of two ducts located in the apparatus and connected to the outside and inside of the barrel. Pressure is generated through one of these ducts by introducing a pressurized inert gas, while the alcoholic beverage sample is extracted through the other duct.

Said sampling device can be used in reverse to introduce the alcoholic beverage or added products into the barrel.

Preferably, and in order to avoid contact with the alcoholic beverage being processed, the thickness of the part of the apparatus introduced into the barrel does not exceed the thickness of the stave of the mouth or bunghole of the barrel.

Also, in another aspect of the invention the at least one sensor of internal environmental conditions and measurement of the partial pressure of dissolved and volatile gases, and the at least one distance sensor may be protected by means of hydrophobic membranes to prevent the sudden rise of the level of alcoholic beverage in the barrel from damaging the sensors. This can happen due to mishandling, such as, for example, introducing the alcoholic beverage into the barrel at a lower temperature than that of the aging and/or maceration room or during barrel transfer.

Advantageously, the apparatus may incorporate an adjusting nut with flaps that allows the apparatus to be pressed into the mouth or bunghole of the barrel by means of one or more blows on the flaps without affecting the apparatus.

Also, advantageously, the apparatus does not need external wiring as it has its own autonomous electrical power source, preferably batteries, and more preferably batteries, and communicates wirelessly with a base station for monitoring the conditions of the alcoholic beverage. The preferred forms of wireless communication are WiFi and LoRa.

Preferably, the part of the apparatus in contact with the alcoholic beverage and the headspace is constructed of food-grade materials and components.

According to another aspect of the invention, the same relates to a system for monitoring the aging and/or maceration processes of an alcoholic beverage in a barrel comprising at least one apparatus, such as the one described above, for monitoring the aging and/or maceration processes of an alcoholic beverage in at least one barrel; the at least one apparatus, connected with a base station so as to record and compare the data of the internal and external environmental conditions, the distance between the at least one apparatus and the alcoholic beverage and the measurement of the partial pressure of dissolved and volatile gases in order to determine the volume of the headspace and the evolution thereof, as well as the partial pressure in the headspace of the at least one barrel of a gas dissolved in the alcoholic beverage and/or the vapor pressure in the headspace of the barrel of a volatile compound of the alcoholic beverage, thus making it possible to determine its concentration.

Each sensor will be programmed to generate specific data packets to be sent to a base station via radio signals on a periodic basis. To avoid excessive power consumption, a hibernation time will be set for each device, depending on the number of measurements per day.

When the sensors are activated after hibernation, they will transmit a data packet to the base station, usually via LoRa or similar protocols, with the frequency band specifications required by the regulations of each country of installation.

The protocol will ensure the transmission of the data to the base station, and the system will enter a new hibernation cycle.

The data entering the base station will be stored in its corresponding database to be processed and represented, preferably based on specific algorithms, thus generating a data set of easy interpretation for the user. Likewise, such data may be assigned to alarms and/or alerts defined by user-specified or predetermined ranges of levels.

The different sensors will have an internal reading and sending cycle to the base station that may be the same or different, depending on the hibernation and acquisition rate determined by the user or by default.

The system according to the invention transfers the raw data from the at least one apparatus to the base station for processing by means of a desktop software which will provide the alarms to the user. In this way, the system allows the measurement of different parameters by means of different algorithms with the full power of the base station of the computer system (software/PC), i.e., the apparatus has sensors that transfer the information to the computer system that interprets these values to determine the conditions of the barrel and the headspace.

The system incorporates a series of alarms and/or alerts that warn the user whenever an undesired situation occurs. These are undesirable situations, by way of example and not limitation:
- The internal or headspace temperature exceeds the external or microenvironment temperature of the barrel.
- The internal or headspace pressure exceeds the external or microenvironment pressure of the barrel.
- The internal or headspace relative humidity must be lower than the external or microenvironment relative humidity of the barrel.
- The SO₂ level is below the established threshold.
- The level of any of the monitoring parameters that the system is monitoring is below or above the set threshold.

As long as none of the undesired situations occurs, the user shall not perform any action on the barrel, or the alcoholic beverage contained therein.

The data transmission between the apparatus and the processor can be wired or wireless. Preferably, the data transmission between the apparatus and the processor is done wirelessly.

In the latter case, the system enables over-the-air *(OTA - over-the-air)* updating, i.e., the wireless delivery of new software, firmware or other data to at least one apparatus comprising the system for reconfiguration, reboot, etc.

Likewise, the control of the environmental conditions of the microenvironment of each barrel through the system's apparatus makes it possible to predict the potential evaporation rate of each zone of the barrel room, and thus analyze the overall volume of the barrel room, and to map it in order to detect differentiated zones and be able to manage potential evaporation in a known way, choosing zones that minimize future shrinkage or changing and/or correcting the environmental conditions of the room through automatic or natural climate controls.

The future shrinkage can be calculated by determining, using the apparatus of the invention, the vapor pressure deficit of water and ethanol, i.e., the difference between saturation and humidity. The vapor pressure of a pure liquid in air depends on the temperature; thus, by measuring the relative humidity and the air temperature, the water vapor pressure deficit of the air can be calculated, and with the temperature the vapor pressure deficit of ethanol can be calculated.

When both deficits are added together, the future shrinkage is obtained.

Preferably, such mapping is done graphically and, even more preferably, in 3D.

The invention relates especially to an apparatus for monitoring the aging and/or maceration processes of an alcoholic beverage in a barrel, as defined in claim 1.

In a second aspect, the invention relates to a system for monitoring the aging and/or maceration processes of an alcoholic beverage in a barrel, as defined in claim 5
In a third aspect, the invention relates to a method for monitoring the aging and/or maceration processes of an alcoholic beverage in a barrel, as defined in claim 8.

In a fourth aspect, the invention relates to an alcoholic beverage obtained according to said method, as defined in claim 10

Other preferred embodiments are described in the dependent claims.

Below, the invention will be described, based on non-limiting examples which illustrate the invention, and which shall not be understood or construed as limiting the scope of the invention.

### Description of the figures

Figure 1 represents the apparatus according to a first embodiment of the invention.
Figure 2A represents section A-A according to FIGURE 1 of the apparatus according to a first embodiment of the invention in which the adjusting nut is in the unscrewed position.

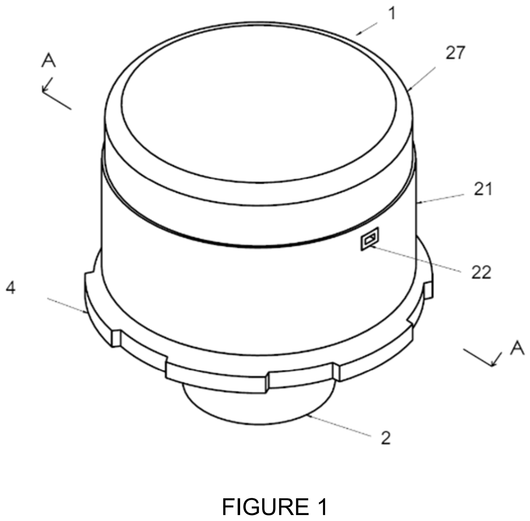

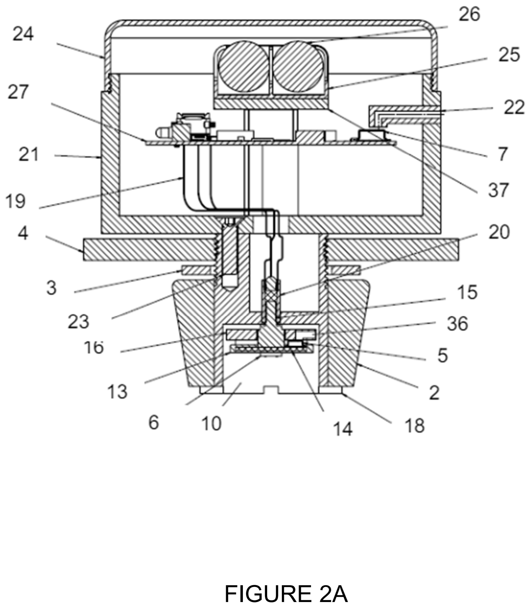

Figure 2A represents section A-A according to FIGURE 1 of the apparatus according to a first embodiment of the invention in which the adjusting nut is in the screwed position acting on the washer and the bung.

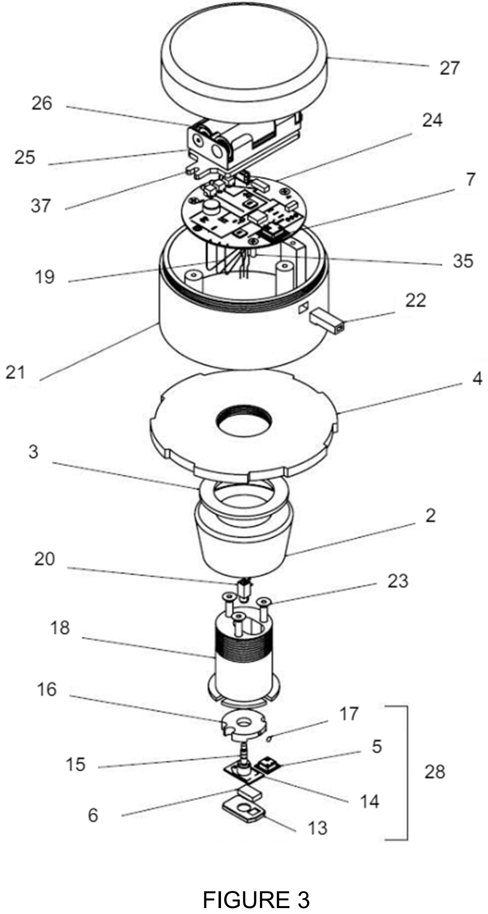

Figure 3 represents an exploded view of the apparatus according to a first embodiment of the invention.

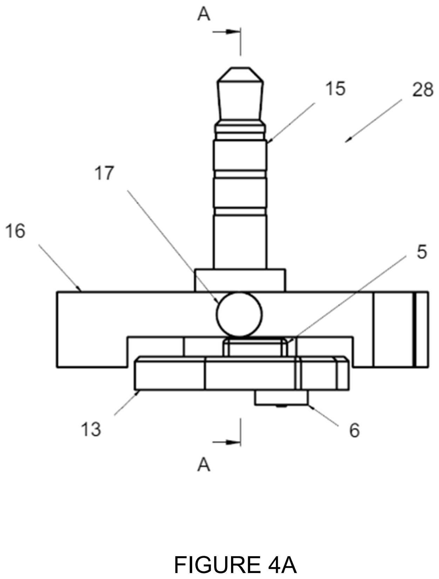

Figure 4A represents the sensor kit of the apparatus according to the invention.

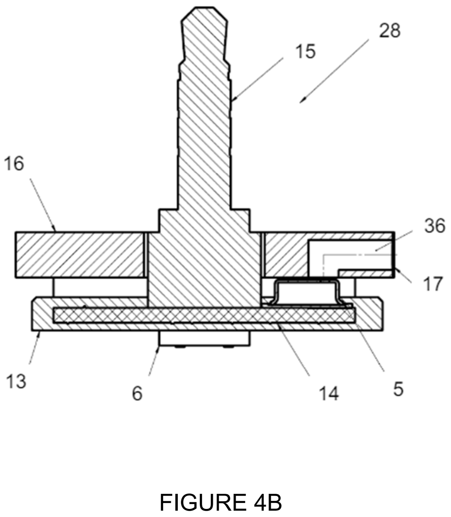

Figure 4B represents section A-A according to FIGURE 4A of the sensor kit of the apparatus according to the invention.

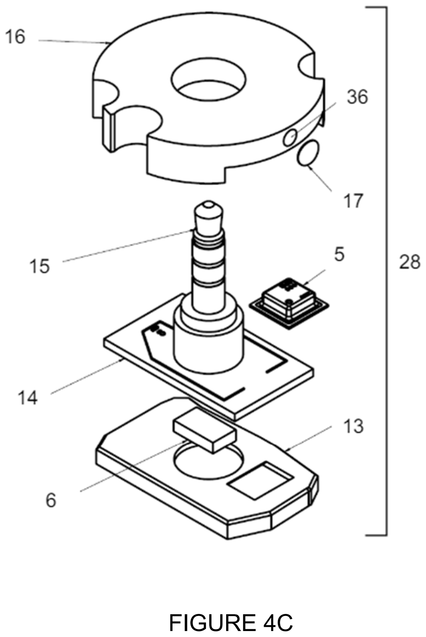

Figure 4C represents an exploded view of the sensor kit of the apparatus according to the invention

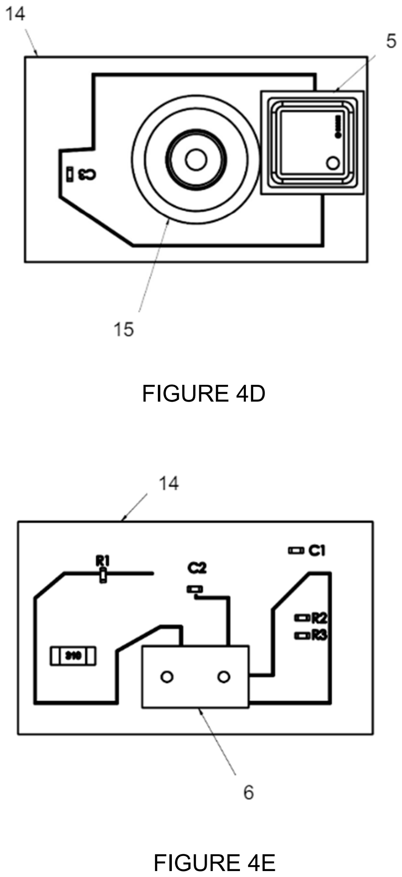

Figure 4D represents the top view of the microchip of the sensor kit of the apparatus according to the invention.
Figure 4E represents the bottom view of the microchip of the sensor kit of the apparatus according to the invention.

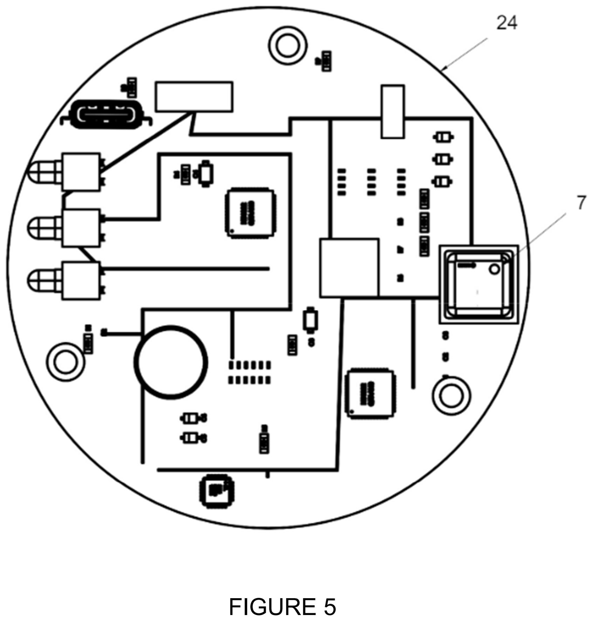

Figure 5 represents the subassembly board or control circuit of the apparatus according to the invention.

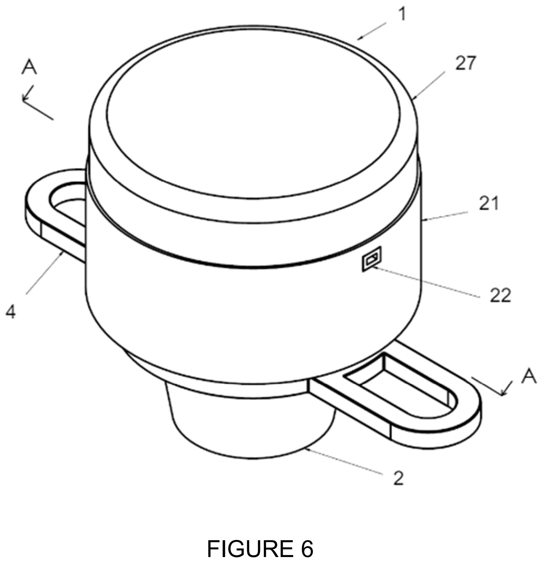

Figure 6 represents the apparatus according to a second embodiment of the invention.

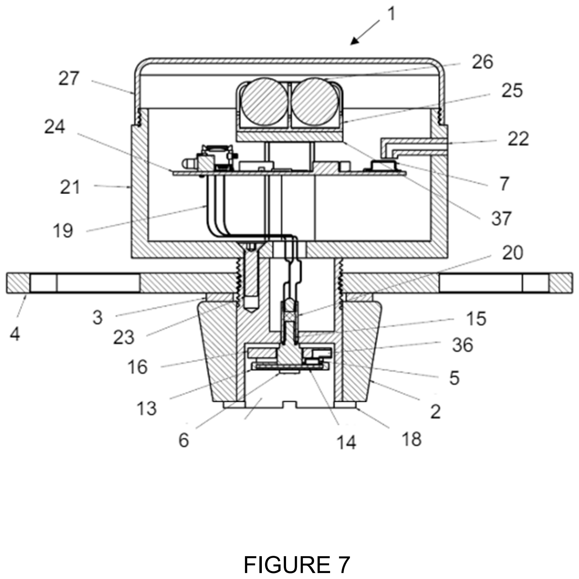

Figure 7 represents section A-A according to FIGURE 6 of the apparatus according to a second embodiment of the invention.

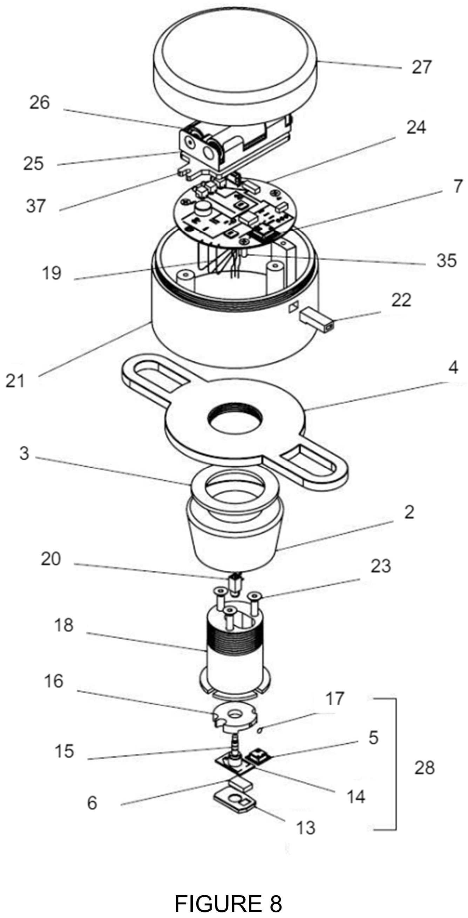

Figure 8 represents an exploded view of the apparatus according to a second embodiment of the invention.

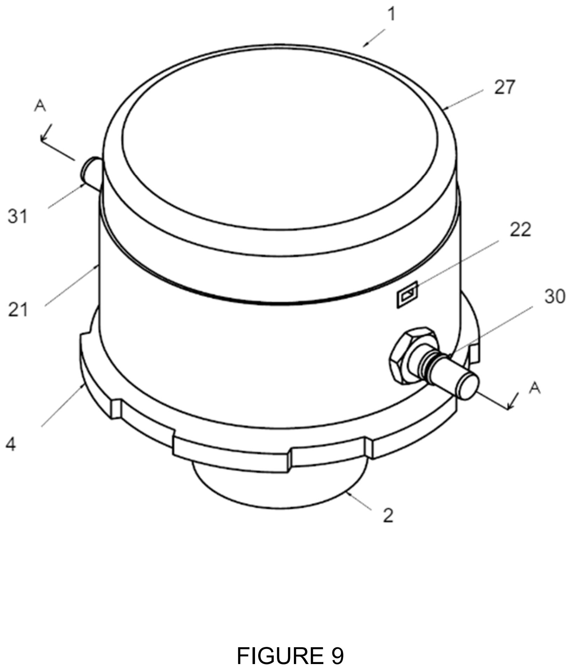

Figure 9 represents the apparatus according to a third embodiment of the invention.

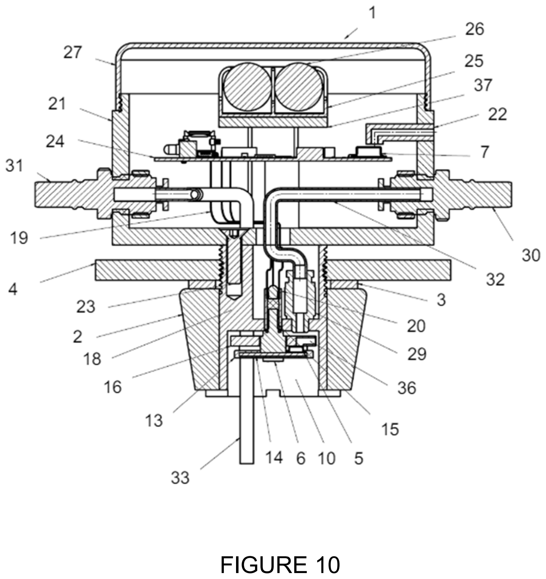

Figure 10 represents section A-A according to FIGURE 9 of the apparatus according to a third embodiment of the invention.

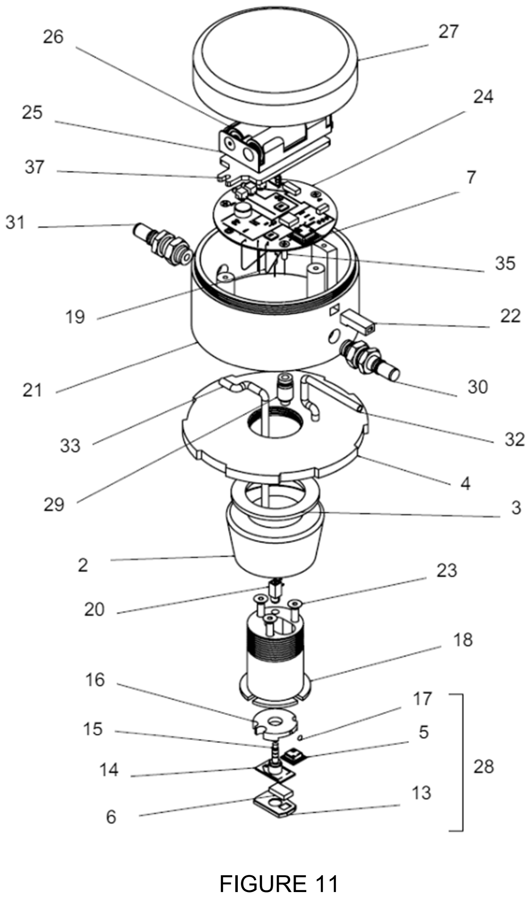

Figure 11 represents an exploded view of the apparatus according to a third embodiment of the invention.

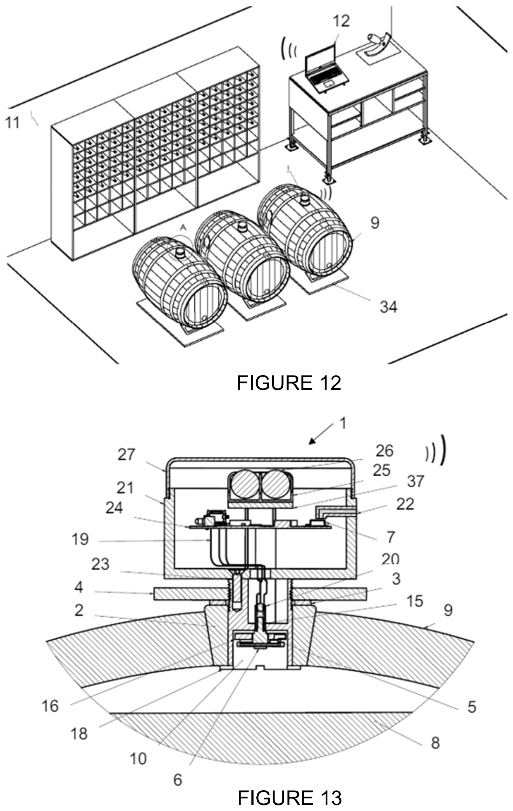

Figure 12 depicts a system for monitoring the aging and/or maceration processes of an alcoholic beverage in a barrel according to the invention.
Figure 13 represents detail A of FIGURE 12 of the apparatus according to the first embodiment located in the bunghole of the barrel.

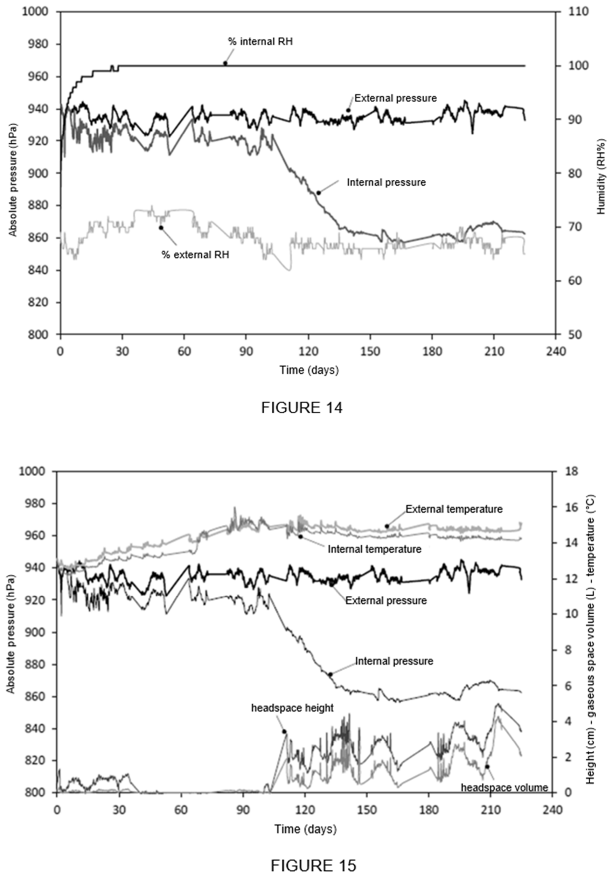

Figure 14 shows an example of the evolution of internal and external relative humidity and internal and external pressure, obtained by monitoring the aging and/or maceration of an alcoholic beverage during eight months.
Figure 15 represents for the example of Figure 14, the evolution of the internal and external temperature and of the internal and external pressure, obtained from the monitoring of the aging and/or maceration of an alcoholic beverage during eight months.

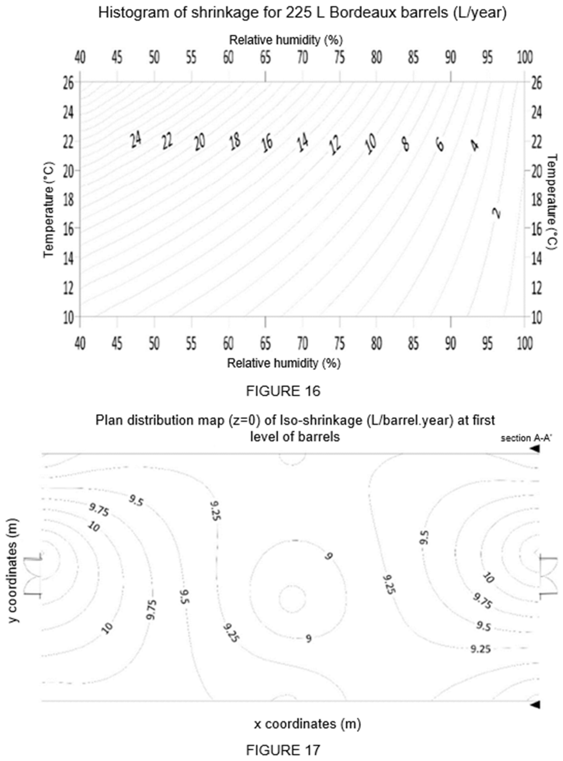

Figure 16 shows the histogram of shrinkage for the barrels for the example shown in Figure 14.
Figure 17 represents, for the example of Figure 14, the distribution map of iso-shrinkage lines at the first level of barrels.

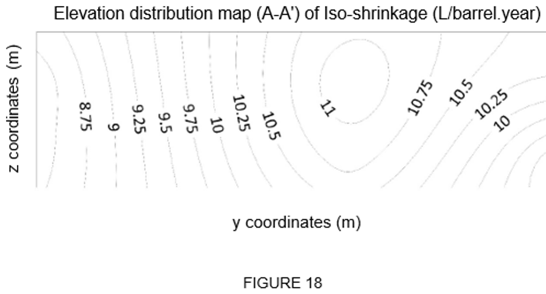

Figure 18 represents, for the example of Figure 14, the elevation distribution map of iso-shrinkage lines.

### References used in the figures:

- 1: Apparatus
- 2: Bung
- 3: Washer
- 4: Adjusting nut
- 5: Internal environmental conditions sensor
- 6: Distance sensor
- 7: External environmental conditions sensor
- 8: Alcoholic beverage
- 9: Barrel
- 10: Headspace
- 11: Barrel room
- 12: Base station
- 13: Protective resin
- 14: Microchip
- 15: Male mini-jack connector
- 16: Minijack holder-distance sensor
- 17: Waterproof membrane
- 18: Body
- 19: Wiring
- 20: Female mini-jack connector
- 21: Housing
- 22: Housing air vent
- 23: Screw
- 24: Subassembly board
- 25: Battery holder
- 26: Batteries
- 27: Housing cover
- 28: Sensor kit
- 29: Sampling fitting
- 30: Sampling injection connector
- 31: Sampling extractor connector
- 32: Sampling gas injector
- 33: Sampling extractor
- 34: Support with load cell
- 35: Screw
- 36: Mini-jack holder air vent

### Preferred embodiment of the invention

With the intention of a better understanding of the characteristics of the invention, below, as examples without limitation, some of the preferred embodiments are described with reference to the accompanying figures. In this regard, the figures are provided for illustrative purposes, and are not intended to be limiting of the present invention. Furthermore, the present invention covers all possible combinations of particular and preferred embodiments indicated herein.

Figure 1 represents the apparatus (1) according to a first embodiment of the invention, showing the lower part of the bung (2) intended to be inserted into the bunghole of the barrel, producing the hermetic seal of the barrel by acting on the adjusting nut (4). In the housing (21) is arranged the air vent (22) intended to transmit to the inside of the apparatus (1) the external environmental conditions so that the external environmental conditions sensor (7) can perform the measurements according to the invention. Above said housing (21) is placed the cover (27) of the apparatus housing (1).

The bung (2) is preferably made of silicone, but it can be made of any other material that provides a good fit with the barrel bunghole (9)
Figure 2A represents section A-A according to FIGURE 1 of the apparatus (1) according to the first embodiment of the invention in such a way that all the components thereof can be observed, of which we highlight first of all, the internal environmental conditions sensor (5), the distance sensor (6) and the external environmental conditions sensor (7). As can be seen, the adjusting nut (4) is in the unscrewed position.

The internal environmental conditions sensor (5) and the distance sensor (6) are arranged on the microchip (14) communicating with the subassembly board (24) through the internal wiring (19).

Preferably, it is proposed as internal environmental conditions sensor (5), the use of the Bosch BME688 sensor, which in addition to measuring the environmental conditions of the internal space of the barrel (barometric pressure, temperature, and relative humidity) has a gas scanner function that can detect volatile organic compounds (VOCs), volatile sulfur compounds (VSCs), and other gases such as carbon monoxide and hydrogen in the parts per billion (ppb) range. The internal environmental conditions sensor (5) can integrate artificial intelligence (AI) and can be trained to detect different substances of interest in the aging and/or maceration of alcoholic beverages, such as SO₂, ethanol, ethyl phenols, acetic acid, ethyl acetate or acetaldehyde among others.

Also, preferably, the distance sensor (6) chosen is the VL53L0X or VL6180, a narrow angle (18-25°) 940 nm time-of-flight *(ToF)* laser measurement module that provides accurate distance measurement independent of target reflectance, which is important in liquid measurement unlike conventional technologies such as ultrasound. It has a resolution of 1 mm with a range of 30 to 1000 mm or 5 to 200 mm, respectively.

The subassembly board (24) comprises an external environmental condition sensor (7). Preferably, it is proposed to use Bosch Sensortech BME280 sensors that measure barometric pressure, temperature, and relative humidity for the external environment. Their knowledge will allow us to know the evaporation rate of water and ethanol, the main components of alcoholic beverages, for example, and of many beverages stored in wooden barrels and barrels of other materials.

**In** one embodiment, the housing (21) is attached to the body (18) by screws (23) and the cover (27) is screwed onto the housing (21)

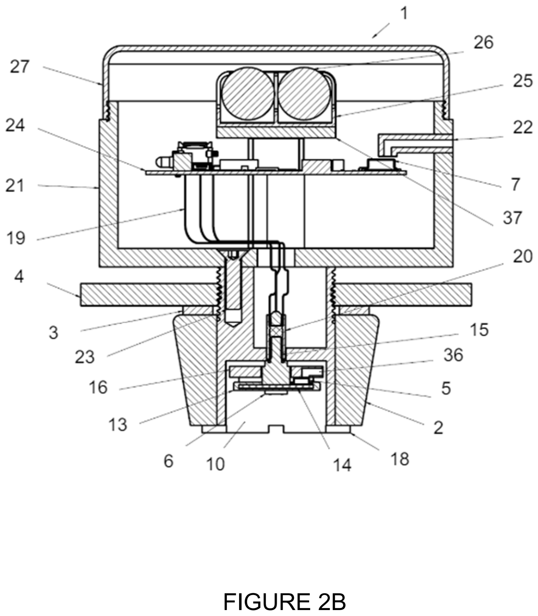

Once the bung (2) of the apparatus (1) is placed in the bunghole of the barrel (9), the adjusting nut (4) is screwed on, manually or with the help of a hand tool, so that the washer (3) presses on the bung (2) producing the hermetic seal of the barrel (9) as shown in Figure 2B, being able, from that moment on, to start monitoring the alcoholic beverage (8), not shown in this figure.

The apparatus (1) has its own autonomous electrical power source, batteries (26), and a WiFi or Lora transmitter/receiver, not referenced in the figures.

This configuration of the apparatus (1) makes it possible to generate the bottom space thereof which becomes, once the apparatus (1) is inserted into the barrel bunghole (9), the headspace (10), for the internal environmental conditions sensor (5) and the distance sensor (6) to perform the appropriate measurements according to the invention.

The sensor kit (28) can also be replaced through this space when any malfunction or anomaly is present.

Figure 3 represents an exploded view of the apparatus according to the first embodiment of the invention, where it can be seen that the subassembly board (24) is placed on some pivots or billets (not referenced in the figures) of the housing (21), the attachment of the plate subassembly (24) and the housing (21) being made by means of the screws (35).

The protective resin (13) in the exploded view is shown separately and as the last component to facilitate its observation, although in reality, said resin is applied on the microchip (14) to protect its components.

Figures 4A-C represent the sensor kit (28) of the apparatus according to the invention containing the internal environmental conditions sensor (5) and the distance sensor (6) in a kit that can be easily and quickly replaced in case it presents anomalies and/or malfunctions. Said sensor kit (28) comprises the internal environmental conditions sensor (5) and the distance sensor (6) located on the microchip (14) to which is attached the male mini-jack connector (15) which, in turn, is attached to the mini-jack holder (16) in which the air vent (36) is made so that the conditions of the headspace (10) easily reach the internal environmental conditions sensor (5). Optionally, this air vent can be protected by the waterproof membrane (17).

The sensor kit (28) is functionally and physically attached to the rest of the components of the apparatus (1) by means of the female mini-jack connector (20) as seen, for example, in Figures 2A-B.

Figure 4D represents the top view of the microchip (14) of the sensor kit (28) of the apparatus (1) according to the invention, in which the internal environmental conditions sensor (5) is observed together with the rest of the electronic components of the microchip (14) and the male mini-jack connector (15).

Figure 4E represents the bottom view of the microchip (14) of the sensor kit (28) of the apparatus according to the invention, in which the distance sensor (6) is observed together with the rest of the electronic components of the microchip (14).

Figure 5 represents the subassembly board (24) of the apparatus (1) according to the invention. This subassembly board (24) contains the microcontroller in charge of managing all the information, as well as the interconnection circuit via radio using the LoRa protocol. This subassembly board (24) houses the place where the power supply of the board itself is placed, as well as USB connectors and three LEDs with programmable light signals; components represented with the standard symbology in this figure. On the circuit surface of the subassembly board (24), there is also the external environmental conditions sensor (7) intended to collect data from the room, such as humidity, barometric pressure, and temperature to be contrasted with the data collected by the internal environmental conditions sensor (5).

Figure 6 represents the apparatus (1) according to a second embodiment of the invention, in which the adjusting nut (4) incorporates flaps to facilitate the screwing of said adjusting nut (4) onto the body (18).

Figure 7 represents section A-A according to FIGURE 6 of the apparatus (1) according to the second embodiment of the invention with the adjusting nut (4) screwed on.

Figure 8 represents an exploded view of the apparatus (1) according to the second embodiment of the invention with the adjusting nut (4) comprising flaps.

Figure 9 represents the apparatus (1) according to a third embodiment of the invention comprising a sampling device. The figure shows both the sampling injection connector (30) through which the gas is injected and the sampling extraction connector (31).

Figure 10 represents section A-A according to FIGURE 9 of the apparatus according to the third embodiment of the invention, showing the sampling gas injector (32) equipped at its end with the fitting (29) preventing the return by said injection, as the sampling extractor (33).

Figure 11 represents an exploded view of the apparatus (1) according to the third embodiment of the invention

Figure 12 depicts a system for monitoring the aging and/or maceration processes of an alcoholic beverage (8) in a barrel (9) according to the invention, in which the apparatus (1) is connected with a base station (12) so as to record and compare data on internal and external environmental conditions, the distance between the apparatus (1) and the alcoholic beverage (8), and the measurement of the partial pressure of dissolved and volatile gases.

The system according to the invention transfers the raw data obtained by the apparatus (1) to the base station (12) for their processing by means of a desktop software which will provide the alarms to the user.

In an example embodiment, the base station (12) comprises a BSEC (Bosch Sensortec Environmental Cluster) software consisting of a library running on the microcontroller of the device for operating the environmental condition sensors (5) and (7), analyzing the data from said sensors (5) and (7), and calculating all outputs thereof such as, for example, environmental humidity or gas scan results. The BSEC software can be configured by means of configuration strings generated during the calibration of each parameter by the BME Al-Studio software

The barrels (9) rest on a support with a load cell (34) which makes it possible to easily check the total loss of the alcoholic beverage (8) contained in each of the barrels (9) by being able to determine at any time and, in particular, at the beginning and end of the aging and/or maceration processes, the weight of the barrel (9) plus alcoholic beverage (8) plus apparatus (1) as a whole.

This system connected with the apparatus (1) allows to know the real quantity of alcoholic beverage (8) evaporated by the environmental conditions of the barrel room (11), since by measuring the variation in weight of the full barrel (9), it discards the alcoholic beverage (8) infiltrated in the wood and allows to know the real loss by evaporation, which in turn depends on the environmental conditions.

Figure 13 represents detail A of FIGURE 12 of the apparatus (1) located in the barrel bunghole (9) in which the headspace (10) can be observed.

Example: Monitoring of aging and/or maceration processes of alcoholic beverages in barrels.

A system was prepared to monitor the aging and/or maceration processes of an alcoholic beverage in a barrel comprising 8 225-liter Bordeaux barrels for monitoring the process.

Eight apparatus for monitoring the aging and/or maceration processes of an alcoholic beverage in a barrel were placed at different points in a large barrel room. In all of them, it was possible to monitor the aging process without intervention, i.e., without having to open the barrel to carry out the usual fillings according to the prior art.

Each of the monitoring apparatus had three sensors, a Bosch BME280 sensor measuring relative humidity, barometric pressure and ambient temperature of the barrel interior; a second sensor measuring headspace height using a VL53L0X narrow angle time-of-flight (ToF) laser measurement module; and a third Bosch BME280 sensor measuring relative humidity, barometric pressure, and ambient temperature of the microenvironment outside the barrel. The eight monitoring apparatus communicated via a WiFi network with a server that collected and stored the measurements in databases and, by means of a database manager or analyzer, the analysis of the monitoring of the different parameters collected by each monitoring apparatus was carried out in a user interface.

Figure 14 shows the evolution of the internal and external relative humidity obtained from the monitoring of the aging process during eight months. It can be seen how after the first few days the headspace saturation (100 % relative humidity) is reached. Saturation that is maintained throughout the aging and/or maceration process, which ensures that the wood remains moist at the top of the barrel and, surprisingly, does not dry out, as was believed in the prior art. In addition, Figure 8 shows how from the first week of aging a depression is generated that is maintained until day 100, at which time a growth of the depression (difference between the external pressure and the internal pressure) begins. The existence of this internal depression guarantees the existence of a hermetic seal of the barrel bung.

Figure 15 also shows the evolution of the internal and external environmental temperature of the barrel, which allows us to verify that there are no exothermic reactions in the alcoholic beverage contained in the barrel and clears the doubt of the possible existence of undesired reactions. Figure 15 also shows the evolution of the height and volume of the headspace, calculated from the geometric shape of a 225-liter Bordeaux barrel and said height. It is clearly seen that only after 100 days of aging does the internal depression increase, and that is when the height/volume of the headspace increases. This is explained by the fact that during the first 100 days, despite the decrease in the liquid contained in the barrel due to the wetting of the wood, the geometric shape of the barrel has been adapting to this decrease in the volume of alcoholic beverages. After that time, the deformation of the staves to adapt reaches its limit and as the alcoholic beverage continues to decrease, the volume of the headspace increases. All this keeps the alcoholic beverage properly thanks to the hermetic seal of the developed apparatus.

In this situation, the total loss of the alcoholic beverage contained in each of the 225-liter barrels, after the aging and/or maceration period of eight months, is significantly lower than when periodic fillings are carried out. Specifically, the average loss during the process was 4.65 liters/barrel, while traditional methods require the addition of up to 22.5 liters/barrel, considering a loss of 11.25 liters/barrel to be admissible.

By monitoring the barrel room of the example described and extrapolating the values obtained, we obtained the distribution maps in plan and elevation of potential iso-shrinkage lines in liters/barrel and year, which allows us to know and optimally use the areas that minimize future shrinkage and even make structural modifications to change and/or correct the environmental conditions of the barrel room through automatic or natural climate controls,

Example: detector based on internal environmental conditions sensor with gas scanner function.

In another example embodiment, the internal environmental conditions sensor with gas scanner function can be used to measure in tanks, or in any container at any time during the preservation/maturation of the alcoholic beverage. Early action makes it possible to treat such a beverage to maintain it at ethyl phenol concentration values well below the perception threshold.

Currently, for example, in model wines, values 15 times lower than the perception thresholds are detected.

A more concrete application of this example embodiment would be to make/modify an air pump corkscrew. The idea is to drill the cork of a bottle by means of the needle, and in the first aspiration to load the corkscrew pump with air (which in normal conditions sucks air from the outside to press the inside of the bottle), and by turning a valve, sucking the gaseous space of the bottle instead of the outside air.

Once aspirated, the corkscrew is blocked and, with the sensor on, the presence of the programmed analyte thresholds can be detected in the gas scanner sensor. This sensor measures, inside the air chamber of the corkscrew, the vapor pressure of the wine analyte with which it was in equilibrium. It also measures the barometric pressure inside the measuring chamber (inside the corkscrew) and together with the temperature and relative humidity of the gaseous space, it allows to correct the programmed thresholds. If the level detected is below the threshold, a green LED will indicate that the bottle is compliant. When the level detected is above the threshold, a red LED will light up indicating that the bottle has a problem and can be returned to the winery.

The invention thus conceived is susceptible of numerous modifications and variations, all of which are within the scope of the appended claims.

In practice, the materials used, as well as the contingent shapes and dimensions, can be any according to the requirements and in accordance with the prior art.

When the technical features mentioned in any claim are followed by reference signs, these reference signs have been included for the sole purpose of increasing the intelligibility of the claims and, accordingly, these reference signs have no limiting effect on the interpretation of each element identified by way of example by these reference signs.

## Claims

1. Apparatus (1) for monitoring the aging and/or maceration processes of an alcoholic beverage (8) in a barrel (9) which is placed in the mouth or bunghole of the barrel, **characterized in that** it comprises a bung (2) and an adjusting nut (4) which hermetically seal the barrel, and at least one internal environmental conditions sensor (5) and one distance sensor (6) both located on the inside and bottom of the apparatus in contact with a headspace (10), and an external environmental conditions sensor (7) of the barrel microenvironment located on the top of the apparatus (1) in contact with the barrel room (11), wherein the internal environmental conditions sensor (5) has a gas scanner function configured to measure the partial pressure in the headspace (10) of a gas dissolved in the alcoholic beverage (8) and/or the vapor pressure in the headspace (10) of a volatile compound of the alcoholic beverage (8), and the thickness of the part of the apparatus (1) inserted into the barrel does not exceed the thickness of the barrel mouth stave or bunghole (9) itself.

2. Apparatus (1) according to the preceding claim, **characterized in that** the internal environmental conditions sensor (5) and the external environmental conditions sensor (7) measure at least one of the following parameters: pressure, temperature and/or relative humidity.

3. Apparatus (1) according to any of the preceding claims, **characterized in that** it comprises specific CO₂, SO₂ and/or REDOX potential sensors configured to obtain the quantitative values of the specific substances/characteristics under control.

4. System for monitoring the aging and/or maceration processes of an alcoholic beverage (8) in at least one barrel (9) comprising at least one apparatus (1) defined in any one of the preceding claims, connected to a base station (12) such that the data obtained by said apparatus (1) are recorded, compared and processed, broadcasting an alarm and/or alert warning the user each time an undesired situation occurs.

5. System for monitoring the aging and/or maceration processes of an alcoholic beverage (8) according to claim 4 **characterized in that** the data entering the base station (12) will be stored in its corresponding database to be processed and represented and assigned to alarms and/or alerts defined by user-specified or predetermined ranges of levels.

6. System for monitoring the aging and/or maceration processes of an alcoholic beverage (8) according to claims 4 and 5, **characterized in that** the control of the environmental conditions of the microenvironment of each barrel (9) allows to predict its potential evaporation rate and thus to analyze globally the volume of the barrel room, and to perform a mapping to detect differentiated zones, and to be able to manage in a known way the potential evaporation.

7. A method for monitoring aging and/or maceration processes of an alcoholic beverage in a barrel comprising the following steps:
a. Providing at least one apparatus (1) as defined in any one of claims 1-4
b. Placing said at least one apparatus in the mouth or bunghole of the barrel (9),
c. Hermetically sealing the at least one barrel (9) with an apparatus (1) comprising a bung (2) and an adjusting nut (4) hermetically sealing the barrel, and at least three sensors, an internal environmental conditions sensor (5), a distance sensor (6), both located on the inside of the apparatus in contact with the headspace (10), and an external environmental conditions sensor (7) of the barrel microenvironment located on the top of the apparatus (1) in contact with the barrel room (11)
d. Connecting the at least one apparatus (1) to a base station (12).
e. Recording, comparing, and processing the data obtained by the apparatus (1),
f. Finishing the aging and/or maceration processes, removing the at least one apparatus (1) and emptying the at least one barrel (8).

8. The method according to claim 7, **characterized in that**, in case an undesired situation occurs, it comprises the following additional steps between e) and f):
a. e.1 Broadcasting an alarm and/or alert that warns the user whenever an undesired situation occurs.
b. e.2 Adding to the alcoholic beverage (8) the amount of product or additive necessary to correct the undesired situation.

9. Aged and/or macerated alcoholic beverage obtained according to the method described in any one of claims 7 to 8.
